(19) Europäisches Patentamt
European Patent Office
Office européen des brevets

(11) EP 1 329 470 B1

(12) # FASCICULE DE BREVET EUROPEEN

(45) Date de publication et mention de la délivrance du brevet:
**01.08.2007 Bulletin 2007/31**

(51) Int Cl.:
***C08G 18/10*** *(2006.01)* ***C08G 18/08*** *(2006.01)*

(21) Numéro de dépôt: **02293006.9**

(22) Date de dépôt: **01.01.2003**

(54) **Polymères associatifs cationiques amphiphiles, procédé de préparation, utilisation comme épaississant et composition les comprenant**

Kationische amphiphile Assoziativpolymere, Verfahren zur Herstellung, Verwendung als Verdickungsmittel und Zusammensetzung

Cationic amphiphilic associative polymers, process for their preparation, use as thickener and composition containing them

(84) Etats contractants désignés:
**AT BE BG CH CY CZ DE DK EE ES FI FR GB GR HU IE IT LI LU MC NL PT SE SI SK TR**

(30) Priorité: **21.01.2002 FR 0200704**

(43) Date de publication de la demande:
**23.07.2003 Bulletin 2003/30**

(73) Titulaire: **L'ORÉAL**
**75008 Paris (FR)**

(72) Inventeurs:
- **Benard, Sylvie**
  **95570 Attainville (FR)**
- **Trouille, Simon**
  **60290 Rantigny (FR)**
- **Arnaud-Roux, Mireille**
  **93600 Aulnay sous Bois (FR)**

(74) Mandataire: **Beetz & Partner**
**Patentanwälte,**
**Steinsdorfstrasse 10**
**80538 München (DE)**

(56) Documents cités:
**EP-A- 0 978 522** **EP-A- 1 174 450**
**US-A- 4 110 286** **US-A- 4 617 341**
**US-A- 5 281 654**

Remarques:
Le dossier contient des informations techniques présentées postérieurement au dépôt de la demande et ne figurant pas dans le présent fascicule.

EP 1 329 470 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention concerne de nouveaux polymères associatifs cationiques amphiphiles, hydrosolubles ou hydrodispersibles, ainsi que leur utilisation dans des compositions pour application topique, notamment à usage cosmétique ou thérapeutique.

Le terme "hydrosoluble" ou "soluble dans l'eau" concernant les polymères de la présente invention signifie que ces polymères ont une solubilité dans l'eau à température ambiante (25°C) au moins égale à 1% en poids, c'est-à-dire que jusqu'à cette concentration, aucun précipité ne peut être détecté à l'oeil nu et la solution est parfaitement limpide et homogène.

On entend par polymères "hydrodispersibles" ou "dispersibles dans l'eau" des polymères qui, lorsqu'on les met en suspension dans l'eau, forment spontanément des globules ayant une taille moyenne, mesurée par diffusion de la lumière sur un appareil de type Coulter, comprise entre 5 nm et 600 nm, et en particulier entre 5 nm et 500 nm.

L'épaississement et/ou la gélification des milieux aqueux par des polymères est depuis longtemps un important sujet de recherche, notamment dans le domaine de la cosmétique et de la pharmacie. L'obtention d'un effet d'épaississement intéressant par un polymère hydrosoluble suppose généralement une masse molaire élevée et un volume hydrodynamique important. La gélification d'un milieu aqueux est alors considérée comme le résultat d'un réseau polymère tridimensionnel obtenu par une réticulation de polymères linéaires ou par copolymérisation de monomères bifonctionnels et polyfonctionnels. L'utilisation de tels polymères de masse molaire très élevée pose cependant un certain nombre de problèmes tels que la texture peu agréable et la difficulté d'étalement des gels obtenus.

Une approche intéressante a consisté à utiliser comme épaississant des polymères capables de s'associer réversiblement entre eux ou avec d'autres molécules ou particules. Cette association physique donne lieu à des systèmes macromoléculaires thixotropes ou rhéofluidifiables, c'est-à-dire des systèmes dont la viscosité dépend des forces de cisaillement auxquelles ils sont soumis.

De tels polymères capables de s'associer réversiblement entre eux ou avec d'autres molécules sont appelés polymères associatifs. Les forces d'interactions mises en jeu peuvent être de nature très différente, par exemple de nature électrostatique, de type liaison hydrogène ou des interactions hydrophobes.

Un cas particulier de polymères associatifs sont des polymères amphiphiles, c'est-à-dire des polymères comportant une ou plusieurs parties hydrophiles qui les rendent solubles dans l'eau et une ou plusieurs zones hydrophobes par lesquelles les polymères interagissent et se rassemblent entre eux ou avec d'autres molécules.

On a déjà préconisé en cosmétique l'utilisation de polymères associatifs et en particulier de polyuréthannes associatifs. Cependant, les propriétés rhéologiques et cosmétiques de ces polymères ne sont pas optimales.

On a découvert une nouvelle famille de polymères amphiphiles associatifs cationiques, hydrosolubles ou hydrodispersibles, dont les qualités épaississantes sont encore améliorées par rapport à l'art antérieur et qui possèdent de bonnes propriétés cosmétiques.

Ses excellentes propriétés épaississantes permettent une utilisation du polymère en quantité réduite. Cet avantage permet une amélioration de la texture de la composition le contenant. Le gel obtenu en utilisant les polymères de l'invention est agréable au toucher et s'étale facilement.

Un objet de la présente invention est un polymère de formule générale (I) suivante :

$$R\text{-}X\text{-}[L\text{-}(Y)_m]_r\text{-}L'\text{-}X'\text{-}R' \qquad (I)$$

dans laquelle :

- R et R', identiques ou différents, représentent un groupement hydrophobe ou un atome d'hydrogène,
- X et X', identiques ou différents, représentent un groupement comportant une fonction amine tertiaire ou quaternaire, portant ou non un groupement hydrophobe,
- L et L', identiques ou différents, représentent un groupement dérivé d'un diisocyanate,
- Y représente un groupement hydrophile ;
- r est un nombre entier compris entre 1 et 100, de préférence entre 1 et 50 et en particulier entre 1 et 25,
- m est un nombre compris entre 1 et 1000,

le polymère contenant au moins une fonction amine protonée ou quaternisée et au moins un groupement hydrophobe, étant entendu que le taux de motif amine quaternisée (ou taux de quaternisation) est d'au moins 85%.

Un autre objet de l'invention est un procédé de préparation des polymères ci-dessus, dans lequel on fait réagir un 'dérivé à amine quaternisée' avec un 'prépolymère diisocyanate'.

Un autre objet de l'invention est l'utilisation d'au moins un polymère tel que défini ci-dessus, comme agent épaississant.

Un autre objet de l'invention est une composition comprenant le polymère tel que défini ci-dessus.

Le terme "polymère" choisi pour désigner les nouveaux polymères associatifs de la présente invention englobe

les polyuréthannes proprement dits, les polyurées et les polythiourées ainsi que des copolymères de ceux-ci.

La famille de polymères associatifs amphiphiles cationiques, hydrosolubles ou hydrodispersibles, conformes à l'invention peut être représentée par la formule générale (I) suivante :

$$R\text{-}X\text{-}[L\text{-}(Y)_m]_r\text{-}L'\text{-}X'\text{-}R' \qquad (I)$$

dans laquelle :

- R et R', identiques ou différents, représentent un groupement hydrophobe ou un atome d'hydrogène,
- X et X', identiques ou différents, représentent un groupement comportant une fonction amine tertiaire ou quaternaire, portant ou non un groupement hydrophobe,
- L et L', identiques ou différents, représentent un groupement dérivé d'un diisocyanate ,
- Y représente un groupement hydrophile ;
- r est un nombre entier compris entre 1 et 100, de préférence entre 1 et 50 et en particulier entre 1 et 25,
- m est un nombre compris entre 1 et 1000,

le polymère contenant au moins une fonction amine protonée ou quaternisée et au moins un groupement hydrophobe, étant entendu que le taux de motif amine quaternisée est d'au moins 85%.

Une famille préférée de polymères associatifs cationiques selon la présente invention est celle pour laquelle le taux de motif amine quaternisée est compris entre 87 et 100%, et préférentiellement compris entre 88 et 99%.

Les fonctions amine quaternisée des polymères résultent de la quaternisation d'une amine tertiaire, par des agents d'alkylation à groupe hydrophobe, c'est-à-dire des composés de type RQ ou R'Q, dans lequel R et R' sont tels que définis plus haut et Q désigne un groupe partant tel qu'un halogénure ou un sulfate.

Les fonctions amines neutralisées des polymères résultent de la neutralisation de l'amine tertiaire ; elles peuvent également résulter de l'hydrolyse de fonctions isocyanate en excès, en bout de chaîne, suivie de l'alkylation des fonctions amine primaire formées, par des agents d'alkylation à groupe hydrophobe, c'est-à-dire des composés de type RQ ou R'Q, dans lequel R et R' sont tels que définis plus haut et Q désigne un groupe partant tel qu'un halogénure ou un sulfate.

Une famille préférée de polymères selon la présente invention est celle correspondant à la formule (I) ci-dessus dans laquelle :

- R et R' représentent tous les deux indépendamment un groupement hydrophobe, et
- X et X' représentent tous les deux indépendamment un groupement comportant une amine quaternaire.

Le groupe hydrophobe du polymère selon l'invention résulte de la réaction de quaternisation; il est introduit par l'agent quaternisant.

La masse moléculaire moyenne en nombre des polymères associatifs amphiphiles cationiques de l'invention est comprise de préférence entre 10 000 et 60 000, en particulier entre 15 000 et 55 000 et idéalement entre 20 000 et 50 000.

Par groupement hydrophobe, on entend un radical ou polymère à chaîne hydrocarbonée, saturée ou non, linéaire ou ramifiée, pouvant contenir un ou plusieurs hétéroatomes tels que P, O, N, S, ou un radical à chaîne perfluorée ou siliconée. Lorsqu'il désigne un radical hydrocarboné, le groupement hydrophobe comporte au moins 10 atomes de carbone, de préférence de 10 à 30 atomes de carbone, en particulier de 12 à 30 atomes de carbone et plus préférentiellement de 18 à 30 atomes de carbone. De préférence, ce groupement hydrophobe est un groupement alkyle, linéaire ou ramifié, en C12-C30 tel qu'un groupement décyle, dodécyle, myristyle, palmityle ou stéaryle.

Préférentiellement, le groupement hydrophobe hydrocarboné provient d'un composé monofonctionnel. A titre d'exemple, le groupement hydrophobe peut être apporté par un agent d'alkylation tel qu'un halogénure ou sulfate d'ammonium comportant un H labile, par exemple une fonction alcool, et une chaîne alkyle en C10-30. Le groupement hydrophobe peut également être un polymère hydrocarboné tel que par exemple le polybutadiène.

Les groupements X et/ou X' peuvent représenter l'une des formules suivantes :

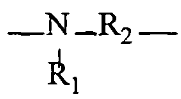 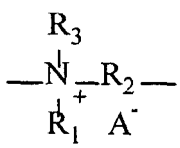 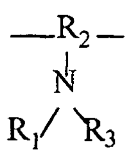 ou 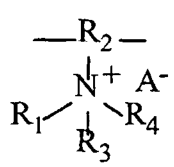 pour X

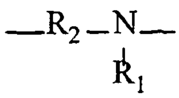 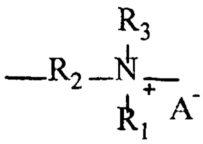 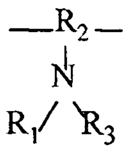 ou 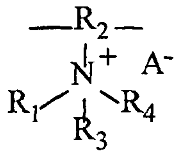 pour X'

dans lesquelles :

$R_2$ représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, P ;
$R_1$, $R_3$ et $R_4$, identiques ou différents, désignent un radical alkyle ou alcényle en $C_1$-$C_{30}$, linéaire ou ramifié, un radical aryle, l'un au moins des atomes de carbone pouvant être remplacé par un hétéroatome choisi parmi N, S, O, P ;
$A^-$ est un contre-ion physiologiquement acceptable.

Les groupements L et L' peuvent représenter un groupe de formule :

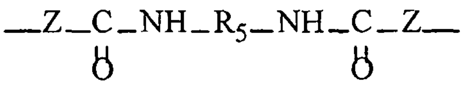

dans laquelle :

Z représente -O-, -S- ou -NH- ; et
$R_5$ représente un radical divalent alkylène ayant de 1 à 20 atomes de carbone, linéaire, ramifié et/ou cyclique, saturé ou insaturé, et pouvant comprendre un ou plusieurs hétéroatomes choisis parmi N, S, O et P.

En ce qui concerne la signification de Y, on entend par groupement hydrophile, un groupement hydrosoluble polymérique ou non. Ce groupement comporte au moins une fonction à hydrogène labile, de préférence au moins deux fonctions à H labile. Ces fonctions à hydrogène labile peuvent être des fonctions alcool, amine primaire ou secondaire ou thiol. Ce composé peut être une molécule ou un polymère terminé aux extrémités des chaînes par l'une de ces fonctions à hydrogène labile.
A titre d'exemple, on peut citer, lorsqu'il ne s'agit pas de polymères, l'éthylèneglycol, le diéthylèneglycol et le propylèneglycol.
Lorsqu'il s'agit, conformément à un mode de réalisation préféré de l'invention, d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Les polymères amphiphiles associatifs cationiques selon l'invention sont généralement formés à partir de diisocyanates et de différents composés possédant des fonctions à hydrogène labile. Les fonctions à hydrogène labile peuvent être des fonctions alcool, amine primaire ou secondaire ou thiol donnant, après réaction avec les fonctions diisocyanate, respectivement des polyuréthannes, des polyurées et des polythiourées.
Les polymères selon l'invention peuvent notamment être obtenus selon un procédé particulier, dans lequel on fait réagir un 'dérivé à amine quaternisée' avec un 'prépolymère diisocyanate'.
Ce procédé permet notamment d'obtenir un taux élevé de quaternisation, en particulier parce que le rendement de la quaternisation est maîtrisé et est rendu indépendant de la masse du polymère.

Le 'prépolymère diisocyanate' peut être obtenu par condensation d'un prépolymère à fonctions à H labile telles que définies ci-dessus avec un diisocyanate de formule : $O=C=N-R_5-N=C=O$
dans lequel $R_5$ est tel que défini plus haut.
L'obtention d'extrémités isocyanates est maîtrisée par le rapport OH/NCO Le rapport préféré est un excès de NCO.
A titre d'exemple de diisocyanate, on peut citer le méthylènediphényl-diisocyanate, le méthylènedicyclohexyl-diisocyanate, l'isophorone-diisocyanate, le toluène-diisocyanate, le naphtalène-diisocyanate, le butane-diisocyanate et l'hexane-diisocyanate.

Le 'dérivé à amine quaternisée' est un composé comportant au moins un motif à fonction amine quaternisée, notamment au moins deux, voire plusieurs motifs à fonction amine. De préférence, ce composé comprend un seul motif à fonction amine. Ce composé comporte également au moins un atome d'hydrogène labile, par exemple deux H labiles, voire plus, porté(s) par exemple par une fonction hydroxyle, amine primaire, amine secondaire ou thiol. On peut également utiliser un mélange de composés monofonctionnels, difonctionnels et/ou multifonctionnels (1, 2 ou plusieurs H labiles); toutefois le pourcentage de composés multifonctionnels est de préférence faible dans le mélange. Préférentiellement le composé est monofonctionnel (un seul H labile).
On peut notamment citer comme 'dérivé à amine quaternisée', le produit de la réaction entre i) un composé choisi parmi la N-méthyléthanolamine, la N-méthyldiéthanolamine, la N,N-diméthyléthanolamine, la N,N-diéthyléthanolamine, la N-tert-butyldiéthanolamine et la N-sulfoéthyldiéthanolamine, et ii) un composé choisi parmi les halogéno-alkyles en C10-30, notamment C12-18, et notamment le bromooctadécane, le chlorooctadécane, le chlorododécyle et le bromododécyle.
Le polymère amphiphile associatif cationique de formule (I) selon la présente invention est soluble ou dispersible dans l'eau et augmente de manière spectaculaire la viscosité de la solution aqueuse dans laquelle il est dissous ou dispersé.
En particulier, le polymère selon l'invention est tel qu'il présente une viscosité $\eta_0$ à 27°C, comprise entre 5 et 100 Pa.s, de préférence entre 5 et 70 Pa.s, et préférentiellement entre 5 et 60 Pa.s, en solution aqueuse à 7% en poids, ladite solution étant préparée de la manière décrite à l'exemple 3 ci-après.
Etant donné ses bonnes propriétés épaississantes et son excellente affinité pour les matières kératiniques, ce type de polymère amphiphile associatif cationique selon l'invention est particulièrement adapté pour la préparation de compositions destinées à un usage cosmétique ou pharmaceutique.
De préférence, le polymère est présent dans les compositions notamment cosmétiques en une quantité comprise entre 0,01-40% en poids de matière sèche, de préférence 0,05-35% en poids, préférentiellement 0,05-30% en poids par rapport au poids de la composition finale.
Les compositions notamment cosmétiques comprenant le polymère selon l'invention peuvent comprendre, dans un milieu cosmétiquement acceptable, les ingrédients usuellement employés dans ce type de composition
Elles peuvent notamment comprendre de l'eau ou un mélange d'eau et d'un solvant tel qu'un alcool en $C_1$-$C_8$, tel que l'éthanol, l'isopropanol, le tert-butanol, le n-butanol; un polyol tel que la glycérine; un glycol comme le butylène glycol, l'isoprène glycol, le propylène glycol, les polyéthylène glycols tels que le PEG-8 ; les éthers de polyol.
Les compositions selon l'invention peuvent également contenir un ou plusieurs additifs tels que des polymères, fixants ou non, anioniques, amphotères, zwitteroniques, non ioniques ou cationiques; des tensioactifs; des agents nacrants; des opacifiants; des solvants organiques; des parfums; des épaississants; des gélifiants; des huiles et/ou des cires d'origine minérale, végétale, animale ou synthétique; des esters d'acides gras; des colorants, des silicones volatiles ou non, organomodifiées ou non, cycliques ou acycliques, ramifiées ou non; des particules minérales ou organiques; des pigments et des charges; des conservateurs; des actifs cosmétiques; des filtres solaires; des agents de stabilisation du pH.
Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.
Les compositions selon l'invention peuvent être utilisées pour le traitement et le soin de la peau du visage et/ou du corps, des muqueuses (lèvres), du cuir chevelu et/ou des cheveux.
Elles trouvent donc une application particulière comme composition de soin du corps ou du visage; composition de nettoyage du corps ou du visage telle que gel-douche, gel de bain, démaquillant; composition de maquillage du corps ou du visage telle que fond de teint, rouge à lèvres, soin pour lèvres, vernis à ongles, soin des ongles, mascara, eye-liner; composition parfumante; composition capillaire telle que composition de coloration des cheveux, composition de déformation permanente des cheveux; composition de protection solaire; composition de nettoyage ou de soin des cheveux telle que shampooing, après-shampooing à rincer ou non, composition à rincer à appliquer avant ou après une coloration, une décoloration, une permanente ou un défrisage ou encore entre les deux étapes d'une permanente ou d'un défrisage; composition capillaire pour le maintien de la coiffure telle qu'une laque, un gel, une mousse ou un spray de coiffage
L'invention est illustrée plus en détail dans les exemples suivants.

**<u>Détermination du taux de quaternisation ou taux de motifs amine quaternisée)</u>**

On détermine la teneur en ions halogénure (bromure ou chlorure) du composé à tester de la manière suivante.
Selon la teneur présumée en halogénures, on prélève la quantité suivante de composé à tester :

| % présumé d'ions halogénure | Prise d'essai (g) |
|---|---|
| 50 | 0,1 |
| 10 | 0,5 |
| 6 | 1,5 |
| 1 | 4 |
| 0,05 | 10 |

On dissout la prise d'essai dans 70 ml d'eau déminéralisée, on ajoute 5 ml de $HNO_3$ (6N), on agite et on titre potentiométriquement avec une solution de nitrate d'argent (0,1N) sous agitation.
De manière analogue, on effectue un essai à blanc, en omettant la prise d'essai et en complétant avec de l'eau distillée pour obtenir le même volume que celui de la solution d'essai.
L'appareillage employé est le suivant :

- mémotitrateur Mettler DL 55
- électrode combinée adaptée au dosage (Mettler DM 141)

La teneur en halogénure (en meq/g) est donnée par la formule suivante :

$$\frac{(Vi - Vo) \times T \times K}{m}$$

dans laquelle :

- Vo : volume versé pour l'essai à blanc (ml)
- Vi : volume versé pour le dosage (ml)
- T : titre de la solution de nitrate d'argent (0,1 M)
- k : facteur de titre de la solution de nitrate d'argent
- m : masse de la prise d'essai (g)

Le taux de quaternisation est égal au rapport entre la teneur mesurée et la teneur théorique pour 100% de quaternisation.

**Exemple 1 : polymère selon l'invention**

Le schéma réactionnel est le suivant :

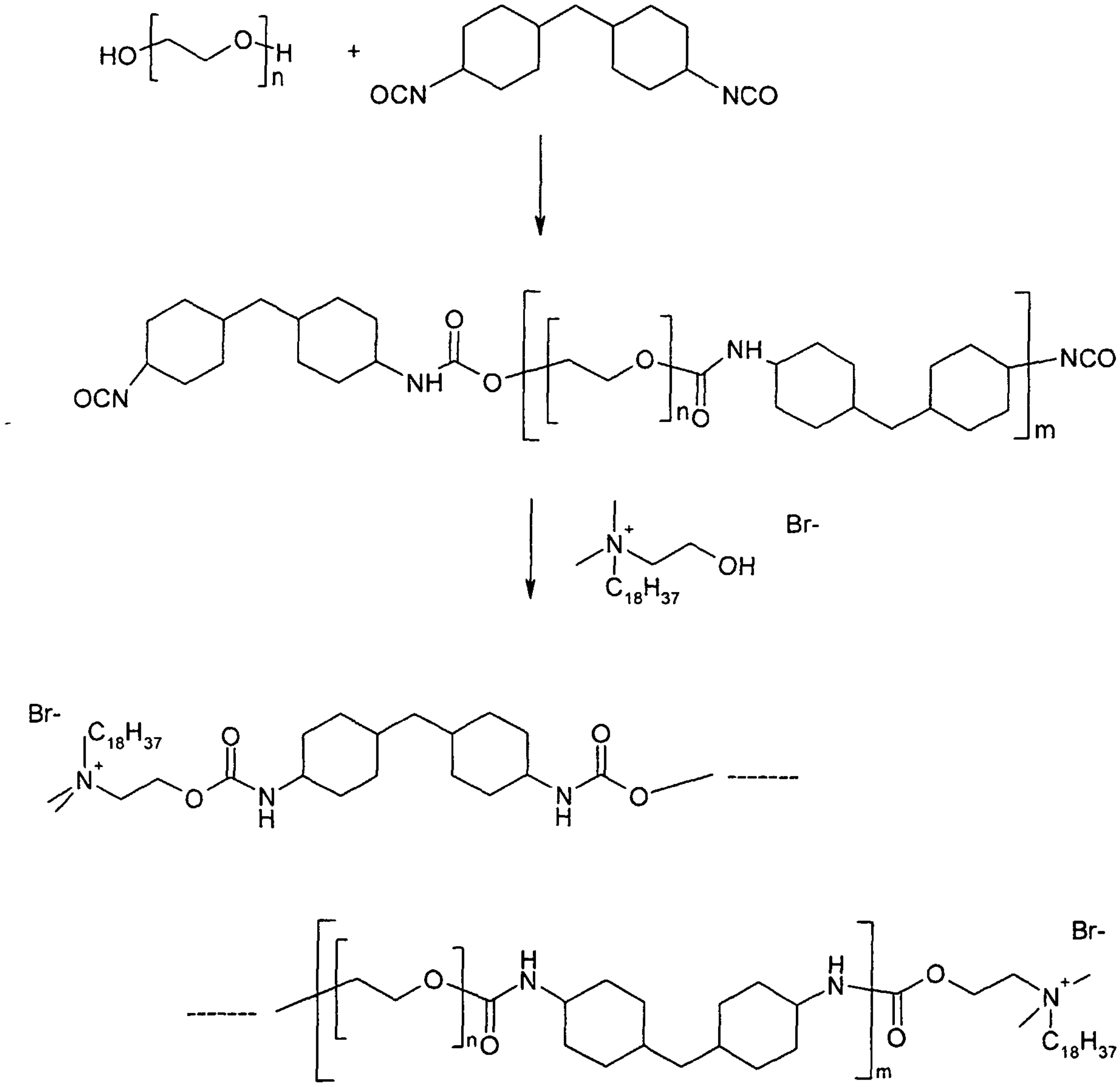

Dans un premier temps, on prépare le bromure de N-stéaryl-N,N-diméthyléthanolammonium par quaternisation de 1 mole de diméthylaminoéthanol avec 1 mole de bromooctadécane, en solution à 50% dans la méthyléthylcétone, au reflux pendant 2 heures. Le produit obtenu est dilué par l'acétone à 20-25°C, filtré, rincé à l'acétone et séché à l'étuve sous vide. On obtient un produit sous forme de cristaux blancs nacrés, quaternisé à 95% (2,30 meq/g; théorie= 2,43 meq/g).

On solubilise 1280 g (0,128 mole) de poly(oxyde d'éthylène) anhydre (Mn 10 000) dans 840 ml de méthyléthylcétone, sous azote. On chauffe au reflux puis on ajoute 60,4 g (0,23 mole) de méthylènedicyclohexyldiisocyanate goutte à goutte et on rince avec 30 ml de méthyléthylcétone. Le milieu réactionnel est chauffé au reflux pendant 8 heures. On ajoute alors 38 g (0,114 mole) de bromure de N-stéaryl-N,N-diméthyléthanolammonium, puis 800 ml de méthyléthylcétone; la concentration du milieu est alors de 52%. On maintient le chauffage à reflux pendant 9 heures.
On ajoute ensuite 2300 ml de méthyléthylcétone, puis on précipite le produit en deux fois, à 70°C, dans 8 litres d'heptane refroidi. Le produit est rincé avec 4 litres d'heptane, filtré sur fritté puis séché sous vide à 40°C pendant 24 heures.
On obtient 1356 g de poudre blanche (rendement 98%).
Le taux de quaternisation est de 91% (0,06 meq/g ; théorie=0,066 meq/g).

<u>**Exemple 2 : polymère comparatif**</u>

| Réactifs: | |
|---|---|
| polyoxyde d'éthylène (PEG) ($M_n$ 10 000) : | 0,010 mole |
| méthylènedicyclohexyldiisocyanate : | 0,018 mole |
| N,N-diméthyléthanolamine : | 0,020 mole |
| bromure de stéaryle : | 0,024 mole |
| octanoate d'étain (catalyseur) : | 0,2 % |

On solubilise 0,010 mole (100 g) de poly(oxyde d'éthylène) ayant une masse moyenne en nombre de 10 000 dans 105 g de THF contenant 0,2% d'octanoate d'étain (catalyseur), puis on ajoute goutte à goutte 0,018 mole (4,71 g) de méthylènedicyclohexyldiisocyanate. On chauffe le milieu réactionnel pendant 15 heures à reflux du THF en ajoutant après 6 heures 100 ml de THF. Pendant la réaction, on observe une disparition partielle de la bande NCO de l'isocyanate en FTIR et l'apparition des bandes CO et NH des liaisons amide formées. Le milieu est très visqueux et transparent.
On ajoute ensuite 0,020 moles (1,78 g) de N,N-diméthyléthanolamine et on laisse la réaction se poursuivre pendant 4 heures au reflux du THF jusqu'à disparition complète de la bande NCO et de la bande OH de l'alcool.
Pour la quaternisation, on ajoute au mélange réactionnel 0,024 mole (8 g) de bromure de stéaryle, c'est-à-dire un excès de 20% en moles par rapport à la N,N-diméthyléthanolamine, puis 100 g de THF pour fluidifier le milieu réactionnel très visqueux. On poursuit le chauffage au reflux du THF pendant 36 heures supplémentaires.
On précipite le polymère obtenu dans de l'éther de pétrole, on filtre et on sèche sous vide à 40°C pendant 24 heures. On obtient ainsi une poudre blanche friable.
On mesure par chromatographie par perméation de gel en milieu aqueux (étalonnage avec polystyrène) une masse moyenne en nombre de 70 000 et une masse moyenne en poids de 115 000, ce qui correspond à un indice de polydispersité de 1,65.
Le taux de quaternisation est de 30% (0,02 meq/g ; théorie=0,066 meq/g).

## Exemple 3 : mesures de viscosité

On prépare une solution aqueuse contenant 7% en poids de polymère dans de l'eau déionisée, à température ambiante sous agitation magnétique d'une durée minimale de 12 heures. On laisse la solution au repos à 27°C pendant au minimum 12 heures avant caractérisation rhéologique.
Les mesures de viscosité sont réalisées en utilisant un rhéomètre à contraintes imposées Haake RS150 équipé d'une géométrie cône - plan de diamètre 35 mm et d'angle 2° à une température de 27°C.
L'échantillon est soumis à une rampe ascendante de contraintes de cisaillement réparties de façon logarithmique entre 0,1 et 1000 Pa. La durée de la rampe est de 2 minutes et 30 secondes.
Les valeurs de viscosités sont déterminées dans la première région newtonienne du rhéogramme, région dans laquelle la viscosité $\eta_0$ ($\eta_0 = \tau / V_c$), parfois aussi appelée viscosité au repos, est constante et indépendante de la contrainte appliquée ($\tau$) ou de la vitesse de cisaillement mesurée ($V_c$).
On obtient les résultats suivants :

- polymère de l'exemple 1 (invention) : $\eta_0 = 17{,}0 \pm 0{,}1$ Pa.s
- polymère de l'exemple 2 (comparatif) : $\eta_0 = 0{,}6 \pm 0{,}1$ Pa.s

## Exemple 4 : polymère selon l'invention

1/ Préparation du bromure de N-lauryl-N,N-diméthyléthanolammonium de formule:

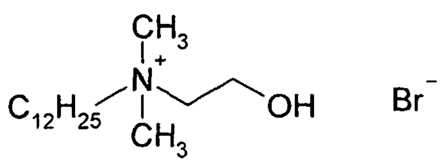

Dans un premier temps, on prépare le bromure de N-lauryl-N,N-diméthyléthanolammonium par quaternisation de 1 mole de diméthylaminoéthanol avec 1 mole de bromododécane, en solution à 50% dans la méthyléthylcétone, au reflux pendant 4 heures. Le produit obtenu est coulé sur 1 litre d'heptane froid, filtré, rincé à l'acétone et séché à 40°C à l'étuve sous vide. On obtient un produit sous forme de cristaux blancs nacrés, quaternisé à 100% (2,96 meq/g).

2/ Préparation du polymère

On solubilise 300 g de poly(oxyde d'éthylène) anhydre (Mn 10 000) dans 300 ml de méthyléthylcétone, sous azote. On chauffe au reflux puis on ajoute 14,15g de méthylènedicyclohexyldiisocyanate goutte à goutte et on rince avec 10 ml de méthyléthylcétone. Le milieu réactionnel est chauffé au reflux pendant $6^{1/2}$ heures.
On ajoute alors 7,1 g de bromure de N-lauryl-N,N-diméthyléthanolammonium, puis 100 ml de méthyléthylcétone. On maintient le chauffage à reflux pendant 8 heures.
On ajoute ensuite 2 litres d'heptane sur le produit refroidi à 60°C, qui précipite.
Le produit précipité est rincé avec 500 ml d'heptane, filtré sur fritté puis séché sous vide à 40°C pendant 24 heures.
On obtient 302 g de poudre blanche (rendement 94%).
Le taux de quaternisation est de 97% (0,064 meq/g ; théorie=0,066 meq/g).

## Revendications

1. Polymère de formule générale (I) suivante :

$$R\text{-}X\text{-}[L\text{-}(Y)_m]_r\text{-}L'\text{-}X'\text{-}R' \qquad (I)$$

dans laquelle :

- R et R', identiques ou différents, représentent un groupement hydrophobe ou un atome d'hydrogène,
- X et X', identiques ou différents, représentent un groupement comportant une fonction amine tertiaire ou quaternaire, portant ou non un groupement hydrophobe,
- L et L', identiques ou différents, représentent un groupement dérivé d'un diisocyanate,
- Y représente un groupement hydrophile ;
- r est un nombre entier compris entre 1 et 100, de préférence entre 1 et 50 et en particulier entre 1 et 25,
- m est un nombre compris entre 1 et 1000,

le polymère contenant au moins une fonction amine protonée ou quaternisée et au moins un groupement hydrophobe, étant entendu que le taux de motif amine quaternisée est d'au moins 85%;
à l'exclusion du polymère ayant un taux de motif amine quaternisée d'au moins 85%, de structure :
$C_{18}H_{37}N^+(CH_3)(CH_3)\text{-}(CH_2)_2\text{-}O\text{-}CONHR_4NHCO\text{-}O(POE)O\text{-}CONHR_4NHCO\text{-}O(CH_2)_2\text{-}N^+(CH_3)(CH_3)C_{l8}H_{37}$
avec $R_4$ = méthylènedicyclohexyle et contre ion = $Cl^-$
et synthétisé à partir des réactifs suivants :

| | |
|---|---|
| Méthylènedicyclohexyldiisocyanate | 2 moles |
| Polyéthylèneglycol | 1 mole |
| N,N-diméthyléthanolamine | 2 moles |
| Agent quatemisant $C_{18}H_{37}OH$ | 2 moles |

2. Polymère selon la revendication 1, ledit polymère ayant une masse moléculaire moyenne en nombre comprise entre 10 000 et 60 000.

3. Polymère de formule générale (1) suivante :

$$R\text{-}X\text{-}[L\text{-}(Y)_m]_r\text{-}L'\text{-}X'\text{-}R' \qquad (I)$$

dans laquelle :

- R et R', identiques ou différents, représentent un groupement hydrophobe ou un atome d'hydrogène,

- X et X', identiques ou différents, représentent un groupement comportant une fonction amine tertiaire ou quaternaire, portant ou non un groupement hydrophobe,
- L et L', identiques ou différents, représentent un groupement dérivé d'un diisocyanate ,
- Y représente un groupement hydrophile;
- r est un nombre entier compris entre 1 et 100, de préférence entre 1 et 50 et en particulier entre 1 et 25,
- m est un nombre compris entre 1 et 1000,

le polymère contenant au moins une fonction amine protonée ou quaternisée et au moins un groupement hydrophobe, étant entendu que le taux de motif amine quaternisée est d'au moins 85%;
ledit polymère ayant une masse moléculaire moyenne en nombre comprise entre 10 000 et 60 000.

**4.** Polymère selon l'une des revendications précédentes, dans lequel le taux de motif amine quaternisée est compris entre 87 et 100%, et préférentiellement compris entre 88 et 99%.

**5.** Polymère selon l'une des revendications précédentes, dans lequel :

- R et R' représentent tous les deux indépendamment un groupement hydrophobe, et
- X et X' représentent tous les deux indépendamment un groupement comportant une amine quaternaire.

**6.** Polymère selon l'une des revendications précédentes, ayant une masse moléculaire moyenne en nombre comprise entre 15 000 et 55 000, et idéalement entre 20 000 et 50 000.

**7.** Polymère selon l'une des revendications précédentes, dans lequel le groupement hydrophobe est un radical ou un polymère à chaîne hydrocarbonée, saturée ou non, linéaire ou ramifiée, pouvant contenir un ou plusieurs hétéroatomes tels que P, O, N, S, ou un radical à chaîne perfluorée ou siliconée.

**8.** Polymère selon l'une des revendications précédentes, dans lequel le groupement hydrophobe est un radical hydrocarboné et comporte au moins 10 atomes de carbone, de préférence de 10 à 30 atomes de carbone, en particulier de 12 à 30 atomes de carbone et plus préférentiellement de 18 à 30 atomes de carbone.

**9.** Polymère selon l'une des revendications précédentes, dans lequel les groupements X et/ou X' représentent l'une des formules suivantes :

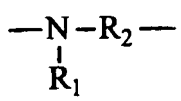 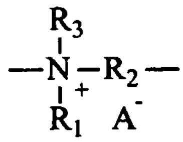 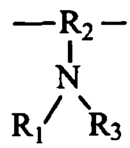 ou 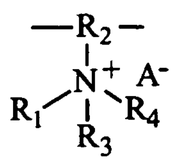

pour X

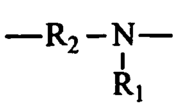 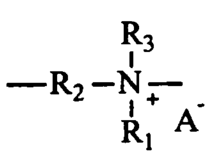 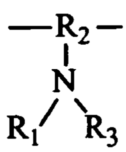 ou 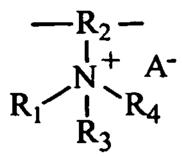

pour X'
dans lesquelles :

$R_2$ représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, P ;

$R_1$, $R_3$ et $R_4$, identiques ou différents, désignent un radical alkyle ou alcényle en $C_1$-$C_{30}$, linéaire ou ramifié, un radical aryle, l'un au moins des atomes de carbone pouvant être remplacé par un hétéroatome choisi parmi N, S, O, P ;
A- est un contre-ion physiologiquement acceptable.

**10.** Polymère selon l'une des revendications précédentes, dans lequel les groupements L et L' représentent un groupe de formule :

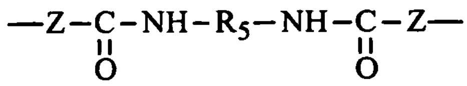

dans laquelle:

Z représente -O-, -S- ou -NH- ; et
$R_5$ représente un radical divalent alkylène ayant de 1 à 20 atomes de carbone, linéaire, ramifié et/ou cyclique, saturé ou insaturé, et pouvant comprendre un ou plusieurs hétéroatomes choisis parmi N, S, O et P.

**11.** Polymère selon l'une des revendications précédentes, dans lequel Y est choisi parmi l'éthylèneglycol, le diéthylèneglycol et le propylèneglycol; les polyéthers, les polyesters sulfonés, les polyamides sulfonés, ou un mélange de ces polymères.

**12.** Polymère selon l'une des revendications précédentes, dans lequel Y est un poly(oxyde d'éthylène) ou un poly(oxyde de propylène).

**13.** Polymère selon l'une des revendications précédentes, tel qu'il présente une viscosité $\eta_0$ à 27°C, comprise entre 5 et 100 Pa.s, de préférence entre 5 et 70 Pa.s, et préférentiellement entre 5 et 60 Pa.s, en solution aqueuse à 7% en poids.

**14.** Procédé de préparation des polymères selon l'une des revendications 1 à 13, dans lequel on fait réagir un 'dérivé à amine quaternisée' avec un 'prépolymère diisocyanate'.

**15.** Utilisation d'au moins un polymère tel que défini selon l'une des revendications 1 à 13, comme agent épaississant.

**16.** Composition comprenant, dans un milieu cosmétiquement acceptable, un polymère de formule générale (1) suivante :

$$R\text{-}X\text{-}[L\text{-}(Y)_m]_r\text{-}L'\text{-}X'\text{-}R' \qquad (I)$$

dans laquelle:

- R et R', identiques ou différents, représentent un groupement hydrophobe ou un atome d'hydrogène,
- X et X', identiques ou différents, représentent un groupement comportant une fonction amine tertiaire ou quaternaire, portant ou non un groupement hydrophobe,
- L et L', identiques ou différents, représentent un groupement dérivé d'un diisocyanate ,
- Y représente un groupement hydrophile ;
- r est un nombre entier compris entre 1 et 100, de préférence entre 1 et 50 et en particulier entre 1 et 25,
- m est un nombre compris entre 1 et 1000,

le polymère contenant au moins une fonction amine protonée ou quaternisée et au moins un groupement hydrophobe, étant entendu que le taux de motif amine quaternisée est d'au moins 85%;
ledit polymère étant présent en une quantité comprise entre 0,01-40% en poids de matière sèche de polymère, par rapport au poids de la composition finale.

**17.** Composition selon la revendication 16, comprenant, dans un milieu cosmétiquement acceptable, le polymère en une quantité comprise entre 0,05-35% en poids de matière sèche, préférentiellement 0,05-30% en poids, par rapport

au poids de la composition finale.

**18.** Composition selon l'une des revendications 16 à 17, dans laquelle le polymère présente un taux de motif amine quaternisée compris entre 87 et 100%, et préférentiellement compris entre 88 et 99%.

**19.** Composition selon l'une des revendications 16 à 18, dans laquelle :

- R et R' représentent tous les deux indépendamment un groupement hydrophobe, et
- X et X' représentent tous les deux indépendamment un groupement comportant une amine quaternaire.

**20.** Composition selon l'une des revendications 16 à 19, dans laquelle le groupement hydrophobe est un radical ou un polymère à chaîne hydrocarbonée, saturée ou non, linéaire ou ramifiée, pouvant contenir un ou plusieurs hétéroatomes tels que P, O, N, S, ou un radical à chaîne perfluorée ou siliconée.

**21.** Composition selon l'une des revendications 16 à 20, dans laquelle le groupement hydrophobe est un radical hydrocarboné et comporte au moins 10 atomes de carbone, de préférence de 10 à 30 atomes de carbone, en particulier de 12 à 30 atomes de carbone et plus préférentiellement de 18 à 30 atomes de carbone.

**22.** Composition selon l'une des revendications 16 à 21, dans laquelle les groupements L et L' représentent un groupe de formule :

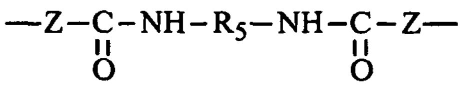

dans laquelle :

Z représente -O-, -S- ou -NH- ; et
$R_5$ représente un radical divalent alkylène ayant de 1 à 20 atomes de carbone, linéaire, ramifié et/ou cyclique, saturé ou insaturé, et pouvant comprendre un ou plusieurs hétéroatomes choisis parmi N, S, O et P.

**23.** Composition selon l'une des revendications 16 à 22, dans laquelle Y est choisi parmi l'éthylèneglycol, le diéthylèneglycol et le propylèneglycol ; les polyéthers, les polyesters sulfonés, les polyamides sulfonés, ou un mélange de ces polymères.

**24.** Composition selon l'une des revendications 16 à 23, dans laquelle le polymère a une masse moléculaire moyenne en nombre comprise entre 10 000 et 60 000; de préférence entre 15 000 et 55 000, et idéalement entre 20 000 et 50 000.

**25.** Composition selon l'une des revendications 16 à 24, se présentant sous la forme d'une composition de soin du corps ou du visage; composition de nettoyage du corps ou du visage telle que gel-douche, gel de bain, démaquillant; composition de maquillage du corps ou du visage telle que fond de teint, rouge à lèvres, soin pour lèvres, vernis à ongles, soin des ongles, mascara, eye-liner; composition parfumante; composition capillaire telle que composition de coloration des cheveux, composition de déformation permanente des cheveux; composition de protection solaire; composition de nettoyage ou de soin des cheveux telle que shampooing, après-shampooing à rincer ou non, composition à rincer à appliquer avant ou après une coloration, une décoloration, une permanente ou un défrisage ou encore entre les deux étapes d'une permanente ou d'un défrisage; composition capillaire pour le maintien de la coiffure telle qu'une laque, un gel, une mousse ou un spray de coiffage.

## Claims

**1.** Polymer of general formula (I) below:

$$R\text{-}X\text{-}[L\text{-}(Y)_m]_r\text{-}L'\text{-}X'\text{-}R' \qquad (I)$$

in which:

- R and R', which may be identical or different, represent a hydrophobic group or a hydrogen atom,
- X and X', which may be identical or different, represent a group comprising a tertiary or quaternary amine function, optionally bearing a hydrophobic group,
- L and L', which may be identical or different, represent a group derived from a diisocyanate,
- Y represents a hydrophilic group;
- r is an integer between 1 and 100, preferably between 1 and 50 and in particular between 1 and 25,
- m is a number between 1 and 1000,

the polymer containing at least one protonated or quaternized amine function and at least one hydrophobic group, it being understood that the content of quaternized amine unit is at least 85%; with the exclusion of the polymer with a content of quaternized amine unit of at least 85%, of structure:
$C_{18}H_{37}N^+ (CH_3) (CH_3)$ - $(CH_2)$-O-CONH$R_4$NHCO-O (POE) 0-CONH$R_4$NHCO-O $(CH_2)_2$-$N^+$(CH3) $(CH_3)$ $C_{18}H_{37}$
with $R_4$ = methylenedicyclohexyl and the counterion = $Cl^-$
and synthesized from the following reagents:

| | |
|---|---|
| methylenedicyclohexyl diisocyanate | 2 mol |
| polyethylene glycol | 1 mol |
| N,N-dimethylethanolamine | 2 mol |
| quaternizing agent $C_{18}H_{37}OH$ | 2 mol. |

2. Polymer according to Claim 1, the said polymer having a number-average molecular mass of between 10 000 and 60 000.

3. Polymer of general formula (I) below:

$$R\text{-}X\text{-}[L\text{-}(Y)_m]_r\text{-}L'\text{-}X'\text{-}R' \qquad (I)$$

in which:

- R and R', which may be identical or different, represent a hydrophobic group or a hydrogen atom,
- X and X', which may be identical or different, represent a group comprising a tertiary or quaternary amine function, optionally bearing a hydrophobic group,
- L and L', which may be identical or different, represent a group derived from a diisocyanate,
- Y represents a hydrophilic group;
- r is an integer between 1 and 100, preferably between 1 and 50 and in particular between 1 and 25,
- m is a number between 1 and 1000,

the polymer containing at least one protonated or quaternized amine function and at least one hydrophobic group, it being understood that the content of quaternized amine unit is at least 85%; the said polymer having a number-average molecular mass of between 10 000 and 60 000.

4. Polymer according to one of the preceding claims, in which the content of quaternized amine unit is between 87% and 100% and preferably between 88% and 99%.

5. Polymer according to one of the preceding claims, in which:

- R and R' both independently represent a hydrophobic group, and
- X and X' both independently represent a group comprising a quaternary amine.

6. Polymer according to one of the preceding claims, having a number-average molecular mass between 15 000 and 55 000 and ideally between 20 000 and 50 000.

7. Polymer according to one of the preceding claims, in which the hydrophobic group is a radical or a polymer containing a linear or branched, saturated or unsaturated hydrocarbon-based chain, which may contain one or more hetero

atoms such as P, O, N and S, or a radical containing a perfluoro or silicone chain.

**8.** Polymer according to one of the preceding claims, in which the hydrophobic group is a hydrocarbon-based radical and contains at least 10 carbon atoms, preferably from 10 to 30 carbon atoms, in particular from 12 to 30 carbon atoms and more preferably from 18 to 30 carbon atoms.

**9.** Polymer according to one of the preceding claims, in which the groups X and/or X' represent one of the following formulae:

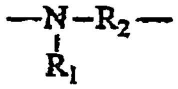
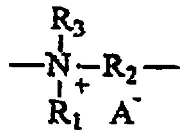
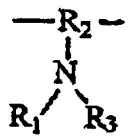
or
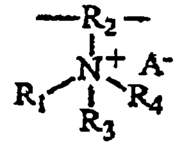

for X

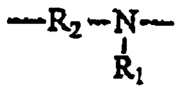
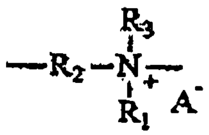
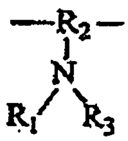
or
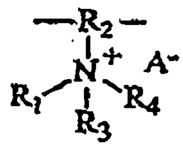

for X'
in which:

$R_2$ represents a linear or branched alkylene radical containing from 1 to 20 carbon atoms, optionally comprising a saturated or unsaturated ring, or an arylene radical, one or more of the carbon atoms possibly being replaced with a hetero atom chosen from N, S, O and P;
$R_1$, $R_3$ and $R_4$, which may be identical or different, denote a linear or branched $C_1$-$C_{30}$ alkyl or alkenyl radical, or an aryl radical, at least one of the carbon atoms possibly being replaced with a hetero atom chosen from N, S, O and P;
$A^-$ is a physiologically acceptable counter-ion.

**10.** Polymer according to one of the preceding claims, in which the groups L and L' represent a group of formula:

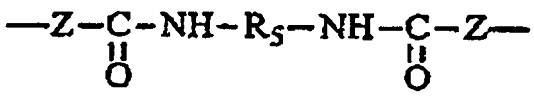

in which:

Z represents -O-, -S- or -NH-; and
$R_5$ represents a linear, branched and/or cyclic, saturated or unsaturated divalent alkylene radical containing from 1 to 20 carbon atoms, which may comprise one or more hetero atoms chosen from N, S, O and P.

**11.** Polymer according to one of the preceding claims, in which Y is chosen from ethylene glycol, diethylene glycol, propylene glycol; polyethers, sulphonated polyesters and sulphonated polyamides, or a mixture of these polymers.

**12.** Polymer according to one of the preceding claims, in which Y is a poly (ethylene oxide) or a poly(propylene oxide).

**13.** Polymer according to one of the preceding claims, such that it has a viscosity $\eta_0$ at 27 °C of between 5 and 100 Pa.s, preferably between 5 and 70 Pa.s and preferentially between 5 and 60 Pa.s, as an aqueous solution at 7% by weight.

**14.** Process for preparing the polymers according to one of Claims 1 to 13, in which a "quaternized amine derivative" is reacted with a "diisocyanate prepolymer".

**15.** Use of at least one polymer as defined according to one of Claims 1 to 13, as a thickener.

**16.** Composition comprising, in a cosmetically acceptable medium, a polymer of general formula (I) below:

$$R\text{-}X\text{-}[L\text{-}(Y)_m]_r\text{-}L'\text{-}X'\text{-}R' \qquad (I)$$

in which:

- R and R', which may be identical or different, represent a hydrophobic group or a hydrogen atom,
- X and X', which may be identical or different, represent a group comprising a tertiary or quaternary amine function, optionally bearing a hydrophobic group,
- L and L', which may be identical or different, represent a group derived from a diisocyanate,
- Y represents a hydrophilic group;
- r is an integer between 1 and 100, preferably between 1 and 50 and in particular between 1 and 25,
- m is a number between 1 and 1000,

the polymer containing at least one protonated or quaternized amine function and at least one hydrophobic group, it being understood that the content of quaternized amine unit is at least 85%;
the said polymer being present in an amount of 0.01-40% by weight of polymer solids relative to the weight of the final composition.

**17.** Composition according to Claim 16, comprising, in a cosmetically acceptable medium, the polymer in an amount of 0.05-35% by weight of solids, preferentially 0.05-30% by weight, relative to the weight of the final composition.

**18.** Composition according to either of Claims 16 and 17, in which the polymer has a content of quaternized amine unit of between 87% and 100% and preferably between 88% and 99%.

**19.** Composition according to one of Claims 16 to 18, in which:

- R and R' both independently represent a hydrophobic group, and
- X and X' both independently represent a group comprising a quaternary amine.

**20.** Composition according to one of Claims 16 to 19, in which the hydrophobic group is a radical or a polymer containing a linear or branched, saturated or unsaturated hydrocarbon-based chain, which may contain one or more hetero atoms such as P, O, N and S, or a radical containing a perfluoro or silicone chain.

**21.** Composition according to one of Claims 16 to 20, in which the hydrophobic group is a hydrocarbon-based radical and contains at least 10 carbon atoms, preferably from 10 to 30 carbon atoms, in particular from 12 to 30 carbon atoms and more preferably from 18 to 30 carbon atoms.

**22.** Composition according to one of Claims 16 to 21, in which the groups L and L' represent a group of formula:

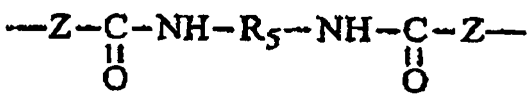

in which:

Z represents -O-, -S- or -NH-; and
$R_5$ represents a linear, branched and/or cyclic, saturated or unsaturated divalent alkylene radical containing from 1 to 20 carbon atoms, which may comprise one or more hetero atoms chosen from N, S, O and P.

**23.** Composition according to one of Claims 16 to 22, in which Y is chosen from ethylene glycol, diethylene glycol, propylene glycol; polyethers, sulphonated polyesters and sulphonated polyamides, or a mixture of these polymers.

**24.** Composition according to one of Claims 16 to 23, in which the polymer has a number-average molecular mass between 10 000 and 60 000, preferably between 15 000 and 55 000 and ideally between 20 000 and 50 000.

**25.** Composition according to one of Claims 16 to 24, which is in the form of a care composition for the body or the face; cleansing composition for the body or the face, such as a shower gel, bath gel or makeup remover; makeup composition for the body or the face, such as foundation, lipstick, lipcare product, nail varnish, nailcare product, mascara or eyeliner; fragrancing composition; hair composition such as hair-colouring composition, permanent-reshaping composition for the hair; antisun composition; cleansing or care composition for the hair such as shampoo, rinse-out or leave-in conditioner, rinse-out composition to be applied before or after dyeing, bleaching, permanent-waving or straightening the hair, or alternatively between the two steps of a permanent-waving or hair-straightening operation; hair composition for holding the hairstyle, such as a styling lacquer, gel, mousse or spray.

**Patentansprüche**

**1.** Polymer der folgenden allgemeinen Formel (I) :

$$R\text{-}X\text{-}[L\text{-}(Y)_m]_r\text{-}L'\text{-}X'\text{-}R' \qquad (I),$$

worin bedeuten:

- R und R', die gleich oder verschieden sind, eine hydrophobe Gruppe oder ein Wasserstoffatom,
- X und X', die gleich oder verschieden sind, eine Gruppe, die eine tertiäre oder quartäre Aminofunktion aufweist, die gegebenenfalls eine hydrophobe Gruppe trägt,
- L und L', die gleich oder verschieden sind, eine von einem Diisocyanat abgeleitete Gruppe,
- Y eine hydrophile Gruppe,
- r eine ganze Zahl im Bereich von 1 bis 100, vorzugsweise 1 bis 50 und insbesondere 1 bis 25,
- m eine Zahl von 1 bis 1 000,

wobei das Polymer mindestens eine protonierte oder quaternisierte Aminofunktion und mindestens eine hydrophobe Gruppe enthält, mit der Maßgabe, dass der Anteil der quaternisierten Aminoeinheit mindestens 85 % beträgt, wobei das Polymer der folgenden Struktur, das die quaternisierte Aminoeinheit in einem Anteil von mindestens 85 % enthält, ausgenommen ist:
$C_{18}H_{37}N+(CH_3)(CH_3)\text{-}(CH_2)_2\text{-}O\text{-}CONHR_4NHCO\text{-}O(PEO)O\text{-}CONHR_4NHCO\text{-}O(CH_2)_2\text{-}N^+(CH_3)(CH_3)C_{18}H_{37}$
mit $R_4$ = Methylendicyclohexyl und das Gegenion = $Cl^-$
das ausgehend von den folgenden Reaktanten synthetisiert wurde:

| | |
|---|---|
| Methylendicyclohexyldiisocyanat | 2 mol |
| Polyethylenglycol | 1 mol |
| N,N-Dimethylethanolamin | 2 mol |
| Quaternisierungsmittel $C_{18}H_{37}OH$ | 2 mol. |

**2.** Polymer nach Anspruch 1, wobei das Polymer eine zahlenmittlere Molmasse von 10 000 bis 60 000 aufweist.

**3.** Polymer der folgenden allgemeinen Formel (I):

$$R\text{-}X\text{-}[L\text{-}(Y)_m]_r\text{-}L'\text{-}X'\text{-}R' \qquad (I),$$

worin bedeuten:

- R und R', die gleich oder verschieden sind, eine hydrophobe Gruppe oder ein Wasserstoffatom,
- X und X', die gleich oder verschieden sind, eine Gruppe, die eine tertiäre oder quartäre Aminofunktion aufweist, die gegebenenfalls eine hydrophobe Gruppe trägt,
- L und L', die gleich oder verschieden sind, eine von einem Diisocyanat abgeleitete Gruppe,
- Y eine hydrophile Gruppe,
- r eine ganze Zahl von 1 bis 100, vorzugsweise 1 bis 50 und insbesondere 1 bis 25,
- m eine Zahl von 1 bis 1 000

wobei das Polymer mindestens eine protonierte oder quaternisierte Aminofunktion und mindestens eine hydrophobe Gruppe enthält, mit der Maßgabe, dass der Anteil der quaternisierten Aminoeinheit mindestens 85 % beträgt; wobei das Polymer eine zahlenmittlere Molmasse von 10 000 bis 60 000 aufweist.

**4.** Polymer nach einem der vorhergehenden Ansprüche, wobei der Anteil der quaternisierten Aminoeinheit im Bereich von 87 bis 100 % und vorzugsweise 88 bis 99 % liegt.

**5.** Polymer nach einem der vohergehenden Ansprüche, worin bedeuten:

- R und R' beide unabhängig voneinander eine hydrophobe Gruppe, und
- X und X' beide unabhängig voneinander eine Gruppe, die ein quartäres Amin aufweist.

**6.** Polymer nach einem der vorhergehenden Ansprüche, das eine zahlenmittlere Molmasse von 15 00 bis 55 000 und idealerweise 20 000 bis 50 000 aufweist.

**7.** Polymer nach einem der vorhergehenden Ansprüche, bei dem die hydrophobe Gruppe eine Gruppe oder ein Polymer mit einer gesättigten oder ungesättigten, linearen oder verzweigten Kohlenwasserstoffkette, die ein oder mehrere Heteroatome enthalten kann, wie P, O, N, S oder eine Gruppe mit perfluorierter Kette oder Siliconkette ist.

**8.** Polymer nach einem der vorhergehenden Ansprüche, wobei die hydrophobe Gruppe eine Kohlenwasserstoffgruppe ist und mindestens 10 Kohlenstoffatome, vorzugsweise 10 bis 30 Kohlenstoffatome, insbesondere 12 bis 30 Kohlenstoffatome und besonders 18 bis 30 Kohlenstoffatome aufweist.

**9.** Polymer nach einem der vorhergehenden Ansprüche, wobei die Gruppen X und/oder X' eine der folgenden Formeln bedeuten:

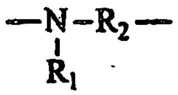
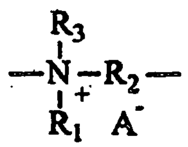
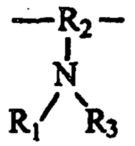
oder
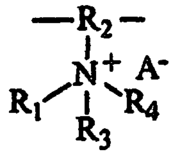

für X

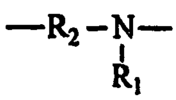
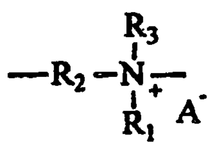
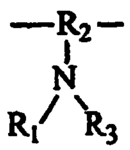
oder
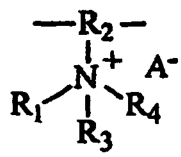

für X'

worin bedeuten:

$R_2$ eine geradkettige oder verzweigte Alkylengruppe mit 1 bis 20 Kohlenstoffatomen, die gegebenenfalls einen gesättigten oder ungesättigten Ring enthält, oder eine Arylengruppe, wobei ein oder mehrere Kohlenstoffatome

durch ein Heteroatom ersetzt sein können, das unter N, S, O, P ausgewählt ist;
$R_1$, $R_3$ und $R_4$, die gleich oder verschieden sind, eine geradkettige oder verzweigte Alkyl- oder Alkenylgruppe mit 1 bis 30 Kohlenstoffatomen, eine Arylgruppe, wobei mindestens ein Kohlenstoffatom durch ein Heteroatom ersetzt sein kann, das unter N, S, O, P ausgewählt ist;
$A^-$ ein physiologisch akzeptables Gegenion.

**10.** Polymer nach einem der vorhergehenden Ansprüche, bei dem die Gruppen L und L' eine Gruppe der folgenden Formel bedeuten:

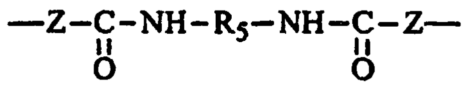

worin bedeuten:

Z -O-, -S- oder -NH-; und
$R_5$ eine zweiwertige Alkylengruppe mit 1 bis 20 Kohlenstoffatomen, die geradkettig, verzweigt und/oder cyclisch, gesättigt oder ungesättigt sein kann und ein oder mehrere Heteroatome enthalten kann, die unter N, S, O und P ausgewählt sind.

**11.** Polymer nach einem der vorhergehenden Ansprüche, wobei Y unter Ethylenglycol, Diethylenglycol und Propylenglycol; Polyethern, sulfonierten Polyestern, sulfonierten Polyamiden oder Gemischen dieser Polymeren ausgewählt ist.

**12.** Polymer nach einem der vorhergehenden Ansprüche, wobei Y ein Poly(ethylenoxid) oder Poly(propylenoxid) ist.

**13.** Polymer nach einem der vorhergehenden Ansprüche, das in einer wässrigen Lösung von 7 Gew.-% bei 27 °C eine Viskosität $\eta_0$ von 5 bis 100 Pa.s, vorzugsweise 5 bis 70 Pa.s und bevorzugt 5 bis 60 Pa.s aufweist.

**14.** Verfahren zur Herstellung von Polymeren nach einem der Ansprüche 1 bis 13, wobei ein "Derivat mit quaternisiertem Amin" mit einem "Diisocyanat-Präpolymer" umgesetzt wird.

**15.** Verwendung mindestens eines Polymers nach einem der Ansprüche 1 bis 13 als Verdickungsmittel.

**16.** Zusammensetzung, die in einem kosmetisch akzeptablen Medium ein Polymer der folgenden allgemeinen Formel (I) enthält:

$$R\text{-}X\text{-}[L\text{-}(Y)_m]_r\text{-}L'\text{-}X'\text{-}R' \qquad (I),$$

worin bedeuten:

- R und R', die gleich oder verschieden sind, eine hydrophobe Gruppe oder ein Wasserstoffatom,
- X und X', die gleich oder verschieden sind, eine Gruppe, die eine tertiäre oder quartäre Aminofunktion aufweist, die gegebenenfalls eine hydrophobe Gruppe trägt,
- L und L', die gleich oder verschieden sind, eine von einem Diisocyanat abgeleitete Gruppe,
- Y eine hydrophile Gruppe,
- r eine ganze Zahl von 1 bis 100, vorzugsweise 1 bis 50 und insbesondere 1 bis 25,
- m eine Zahl von 1 bis 1 000,

wobei das Polymer mindestens eine protonierte oder quaternisierte Aminofunktion und mindestens eine hydrophobe Gruppe enthält, mit der Maßgabe, dass der Anteil der quaternisierten Aminoeinheit mindestens 85 % beträgt;

wobei das Polymer in einer Menge von 0,01 bis 40 Gew.-% Trockensubstanz, bezogen auf das Gesamtgewicht der fertigen Zusammensetzung, enthalten ist.

**17.** Zusammensetzung nach Anspruch 16, die in einem kosmetisch akzeptablen Medium das Polymer in einer Menge

von 0,05 bis 35 Gew.-% Trockensubstanz, vorzugsweise 0,05 bis 30 Gew.-%, bezogen auf das Gesamtgewicht der fertigen Zusammensetzung, enthält.

**18.** Zusammensetzung nach einem der Ansprüche 16 bis 17, wobei das Polymer einen Anteil an quaternisierter Amineinheit von 87 bis 100 % und vorzugsweise 88 bis 99 % enthält.

**19.** Zusammensetzung nach einem der Ansprüche 16 bis 18, worin bedeuten:

- R und R' alle beide unabhängig voneinander einer hydrophobe Gruppe und
- X und X' beide unabhängig voneinander eine Gruppe, die ein quartäres Amin enthält.

**20.** Zusammensetzung nach einem der Ansprüche 16 bis 19, wobei die hydrophobe Gruppe eine Gruppe oder ein Polymer mit einer gesättigten oder ungesättigten, linearen oder verzweigten Kohlenwasserstoffkette ist, die ein oder mehrere Heteroatome, wie P, O, N, S, enthalten kann, oder eine Gruppe mit perfluorierter Kette oder Siliconkette.

**21.** Zusammensetzung nach einem der Ansprüche 16 bis 20, wobei die hydrophobe Gruppe eine Kohlenwasserstoffgruppe ist und mindestens 10 Kohlenstoffatome, vorzugsweise 10 bis 30 Kohlenstoffatome, insbesondere 12 bis 30 Kohlenstoffatome und besonders bevorzugt 18 bis 30 Kohlenstoffatome aufweist.

**22.** Zusammensetzung nach einem der Ansprüche 16 bis 21, wobei die Gruppen L und L' eine Gruppe der folgenden Formel bedeuten:

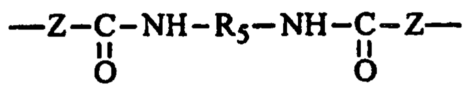

worin bedeuten:

Z -O-, -S- oder -NH-; und
$R_5$ eine zweiwertige, geradkettige, verzweigte und/oder cyclische, gesättigte oder ungesättigte Alkylengruppe mit 1 bis 20 Kohlenstoffatomen, die ein oder mehrere Heteroatome enthalten kann, die unter N, S, O und P ausgewählt sind.

**23.** Zusammensetzung nach einem der Ansprüche 16 bis 22, wobei Y unter Ethylenglycol, Diethylenglycol und Propylenglycol; Polyethern, sulfonierten Polyestern, sulfonierten Polyamiden oder Gemischen dieser Polymere ausgewählt ist.

**24.** Zusammensetzung nach einem der Ansprüche 16 bis 23, wobei das Polymer eine zahlenmittlere Molmasse von 10 000 bis 60 000, vorzugsweise 15 000 bis 55 000 und idealerweise 20 000 bis 50 000 aufweist.

**25.** Zusammensetzung nach einem der Ansprüche 16 bis 24, die als Zusammensetzung für die Pflege des Körpers oder Gesichts; Zusammensetzung für die Reinigung des Körpers oder des Gesichts, beispielsweise als Duschgel, Badegel, Abschminkmittel; Zusammensetzung zum Schminken des Körpers oder des Gesichts, wie als Make-up, Lippenstift, Lippenpflege, Nagellack, Nagelpflege, Mascara, Eyeliner; parfümierende Zusammensetzung; Zusammensetzung für die Haarbehandlung, beispielsweise als Zusammensetzung zum Färben der Haare, Zusammensetzung, um Haare dauerhaft zu verformen; Zusammensetzung zum Sonnenschutz; Zusammensetzung für die Reinigung oder die Pflege der Haare, wie als Haarwaschmittel, Konditioner, der ausgespült wird oder im Haar verbleibt, Zusammensetzung, die ausgespült wird und vor oder nach einer Färbung, Entfärbung, dauerhaften Verformung oder Entkräuselung oder auch zwischen den beiden Schritten einer dauerhaften Verformung oder Entkräuselung aufgebracht wird; Zusammensetzung für die Haarbehandlung zur Festigung der Frisur, wie als Lack, Gel, Schaum oder Spray für die Frisur, vorliegt.